# EUROPEAN PATENT APPLICATION

(11) **EP 1 361 434 A1**
(43) Date of publication of application: **12.11.2003**
(21) Application number: 02729544.3
(22) Date of filing: 11.01.2002
(51) Int. Cl.: G01N 33/53, G01N 33/566, G01N 21/64, G01N 21/27, G01N 37/00

(54) **SELECTIVELY HYBRIDIZABLE SUBSTANCE IMMOBILIZATION FIBER, FIBER ARRAY COMPSIRING BUNDLE OF SUCH FIBERS, SELECTIVE HYBRIDIZING METHOD, DEVICE THEREFOR, AND BASE**

(30) Priority: 12.01.2001 JP 2001005775
(71) Applicant: TORAY INDUSTRIES, INC., Tokyo 103-8666 (JP)
(72) Inventor: SONE, Saburo, Totsuka-ku, Yokohama-shi, Kanagawa 244-0 (JP); NAGINO, Kunihisa, Moriyama-shi, Shiga 524-0021 (JP); HIGASA, Masashi, Takatsuki-shi, Osaka 569-1115 (JP); NOBUMASA, Hitoshi, Otsu-shi, Shiga 520-0043 (JP); WATANABE, Koji, Kusatsu-shi, Shiga 525-0057 (JP)
(74) Representative: Coleiro, Raymond
(86) International application number: JP0200115
(87) International publication number: WO02056011

(57) **Abstract**

Disclosed is an array immobilizing selective binding substances thereon, with which each sample can be distinguished without relying on the positions, an arbitrary array may be made, the density may be freely changed depending on the method of using, and with which the reaction between the bound sample and another sample can be detected easily. The present invention provides a fiber on which a selective-binding substance is immobilized, or a fiber array comprising a bundle of the fibers. A method for binding reaction between a selective binding substance and a corresponding selective binding substance, in which a test sample and/or the corresponding selective binding substance is(are) moved relative to the surface on which the selective binding substance is immobilized by, for example, applying an AC voltage in a direction crossing the perpendicular axis of the surface on which the selective binding substance is immobilized was also provided.

## Description

### Technical Field

The present invention relates to a fiber on which a substance that selectively binds to a test substance (referred to as "selective binding substance" in the present specification), and a fiber array containing a bundle of the fibers, and method for selective binding reaction between the selective binding substance and a corresponding selective binding substance which selectively binds thereto, and apparatus and substrate therefor.

### Background Art

Studies of analysis of genetic information of various organisms have been started, and a number of genes including human genes and nucleotide sequences thereof, and information about proteins encoded by the genes and sugar chains secondarily prepared from the proteins are now rapidly disclosed. Functions of polymers such as genes of which sequences have been clarified, proteins and sugar chains may be investigated by various methods. As for main methods for nucleic acids, the relationships between various genes and their expressed functions in the body may be investigated utilizing complementarity between various nucleic acids/nucleic acids, by a method such as Northern hybridization or Southern hybridization. As for proteins, the functions and expression of proteins may be investigated utilizing protein/protein reactions, as represented by Western hybridization.

In recent years, new methods or methodology called DNA microarray method (DNA chip method) has been developed as methods for analyzing expression of a number of genes at one time, and the methods are drawing attention. The principle of these methods are the same as that of the conventional methods in the respect that they are methods for detecting and quantifying nucleic acids utilizing nucleic acid/nucleic acid hybridization. These methods may also be applied to the detection and quantification of proteins and sugar chains based on the binding reactions between protein/protein, between sugar chain/sugar chain and between sugar chain/protein. These technologies are mainly characterized in that a number of DNA fragment, proteins or sugar chains are immobilized in an array on a planar substrate called microarray or chip. As for the concrete method for using the microarray, for example, genes expressed in the cells of interest are labeled with a fluorescent dye to prepare a sample, the sample is applied to the planar substrate so as to allow binding between complementary nucleic acids (DNA or RNA), the site is labeled with a fluorescent dye or the like and then the positions are read at a high speed using a high resolution analyzer, or the response based on an electrochemical reaction such as electric current or the like is detected. By these methods, the types of genes contained in the sample may be quickly identified.

As for the technique for immobilizing a nucleic acid on the substrate, methods in which poly-L-lysine, aminosilane or the like is coated on the substrate made of a glass or the like, and the nucleic acids are immobilized thereon, and the like have been developed to further increase the density, in addition to the methods in which the nucleic acids are immobilized at a high density on a Nylon sheet or the like in the same manner as in the above-mentioned Northern method,

However, it is pointed out that by the methods in which nucleic acids are spotted on a substrate prepared by chemically or physically modifying a solid surface such as glass, the density of the spots and the amount of the nucleic acid which can be immobilized per one spot are small, and the reproducibility is poor. Further, it is said that a large cost down per one chip is difficult due to an expensive production apparatus and production process including a number of steps. Further, since the distinguishment between the nucleic acids must be relied on the positions of the spots, so that the arrangement and distinguishment of the spots on the substrate are not simple.

As for the microarray using proteins and sugar chains, the same problems as those using nucleic acids exist

As a method which does not utilize the planar substrate, methods utilizing microbeads are known. However, these methods are incomplete in the distinguishment of the various types of microbeads each on which the respective nucleic acid, protein or sugar chain is immobilized, and it is difficult to prepare one in which the designated compounds are arrayed with a designated sequence with good reproducibility.

On the other hand, it has been tried to immobilize nucleic acids in hollow fibers (EP-A-1162262). According to this technique, by immobilizing a nucleic acid in one hollow fiber so as to make a relationship such that one nucleic acid corresponds to one fiber, each of the nucleic acids is distinguished, However, with this technique, the hollow fibers are finally bundled, and each nucleic acid is recognized based on the positional relationship of the fibers, so that the problem in the method in which the nucleic acids are spotted on a substrate is not overcome.

Since the nucleic acid solutions used for nucleic acid/nucleic acid hybridization are valuable, it is desired to carry out the hybridization reaction using the nucleic acids in amounts as small as possible. To this end, it is thought to decrease the concentrations of the nucleic acids in the nucleic acid solutions. To attain a good efficiency of hybridization even when the concentrations of the nucleic acid solutions are small, it is tried to promote the efficiency of hybridization by arranging the specimen nucleic acids-immobilized sites on a substrate of the above-mentioned microarray, which substrate has an electrically conductive layer; generating electric field by applying positive potential to the specimen nucleic acids-immobilized sites, thereby attracting the sample nucleic acids in the nucleic acid solution to the vicinity of the specimen nucleic acid-immobilized sites so as to increase the local nucleic acid concentration in the vicinity of the specimen nucleic acid-immobilized sites (Japanese Laid-open Patent Application (Kokai) No. 8-154656).

As for microarrays using proteins or sugar chains, the effect similar to the microarrays using nucleic acids is expected.

However, the effect to promote measurement sensitivity or to shorten the hybridization time are not satisfactory, so that a more effective hybridization method is demanded.

### Disclosure of the Invention

Under these circumstances, establishment of a new systematic methodology by which macromolecules may be immobilized to a prescribed concentration and may be arrayed in a measurable manner at a high density with a good reproducibility, and by which each of the macromolecules may be recognized without relying on the positions thereof, and which may be applied to inexpensive mass production is strongly demanded for the analysis of macromolecules, that is thought to increase its importance. This is an object of the present invention.

More particularly, an object of the present invention is to provide means by which each sample may be distinguished without relying on the positions, an arbitrary array may be made, the density may be freely changed depending on the method of using, and by which the reaction between the bound sample and another sample can be detected easily, when compared with the process for producing macromolecule array by microspotting or micro-pouring on a two-dimensional carrier such as nylon sheet or glass.

Further, establishment of a method for selective binding reaction, by which valuable macromolecule samples such as nucleic acids, proteins, sugar chains, antibodies and antigens may be utilized in small amounts and effectively is strongly demanded for the analysis of macromolecules, that is thought to increase its importance. This is also an object of the present invention.

More particularly, with the conventional microarrays such as those in which a number of selective binding substances such as DNA fragments, proteins or sugar chains are immobilized in an array at a high density; those prepared by bundling the porous hollow fibers in which the selective binding substances are immobilized, cutting the resultant in the direction crossing the fiber axis to prepare thin pieces to constitute a two-dimensional high density fiber array in which the selective binding substances are immobilized on the surfaces of the fibers; or those in which the selective binding substances are immobilized on the surfaces of arrayed fibers, and the fibers are arrayed in a three-dimensional structure; the hybridization reaction is relied on the natural diffusion of the selective binding substance, so that it is difficult to cause the hybridization reaction effectively using a solution containing small amounts of selective binding substances, and to effectively utilize the valuable selective binding substances. Even with the method for effectively carrying out the hybridization reaction of the selective binding substances utilizing electric attraction, which was invented for overcoming the inefficiency of the conventional hybridization methods, the promotion of efficiency is not sufficient.

In view of this, an object of the present invention is to provide a method for selective binding reaction, which overcomes the above-mentioned drawbacks of the prior art and which causes the selective binding reaction effectively utilizing small amounts of selective binding substances, and to provide an apparatus and substrate therefor.

The present inventors intensively studied to abolish the conventional concept by which the arraying process of the selective binding substances and the immobilization process are conducted on the two-dimensional carrier, and to discover that a fiber bundle may be provided, with which each fiber therein on which each sample is immobilized is distinguished without relaying on its position in a three-dimensional structure, by conducting the immobilization process independently on a one-dimensional fiber (on one fiber) which can be distinguished based on the support, magnetism, bar code, color or shape.

Further, the present inventors discovered that a fiber or array of fibers with which the substance reacted with the selective binding substance immobilized on the fiber can be directly detected may be prepared, by using a light-transmitting material or electrically conductive material for constituting the fibers, thereby completing the present invention.

That is, the present invention provides a fiber on which a selective-binding substance is immobilized, or a fiber array comprising a bundle of the fibers.

The present inventors further intensively studied for attaining the above-mentioned object to discover that the efficiency of the hybridization reaction may be promoted by increasing the probability of collision between the selective binding substance and the corresponding selective binding substance by moving the corresponding selective binding substance in the vicinity of the selective binding substance immobilized on the microarray substrate or on the fiber, thereby completing the present invention.

That is, the present invention provides a method for binding reaction between a selective binding substance and a corresponding selective binding substance, comprising immobilizing the selective binding substance on a substrate; and making a test sample contacted to the immobilized selective binding substance, which test sample contains the corresponding selective binding substance which selectively binds to the selective binding substance, thereby carrying out the binding reaction; the test sample and/or the corresponding selective binding substance being moved relative to the surface on which the selective binding substance is immobilized, in the step of immobilizing the selective binding substance on a substrate and making a test sample contacted to the immobilized selective binding substance. The present invention also provides an apparatus for conducting a binding reaction including a step of immobilizing the selective binding substance on a substrate and making a test sample contacted to the immobilized selective binding substance, which test sample contains the corresponding selective binding substance which selectively binds to the selective binding substance, thereby carrying out the binding reaction, the apparatus comprising a base on which the substrate is mounted; electrodes arranged in a direction crossing the perpendicular axis of the surface on which the selective binding substance is immobilized, and arranged outside the both ends of the area on which said selective binding substance is immobilized; and means for applying AC voltage between the electrodes. The present invention still further provides a substrate for conducting a binding reaction including a step of immobilizing the selective binding substance on the substrate and making a test sample contacted to the immobilized selective binding substance, which test sample contains the corresponding selective binding substance which selectively binds to the selective binding substance, thereby carrying out the binding reaction, the substrate comprising a site for immobilizing the selective binding substance; and electrodes arranged outside the both ends of region for immobilizing the selective binding substance.

By the present invention, a fiber on which a selective-binding substance is immobilized, and a fiber array comprising a bundle of the fibers are provided. By the present invention, a fiber on which a selective binding substance is immobilized efficiently with a good reproducibility is provided. Further, by combining these fibers to form a fiber array, a selective binding substance-immobilized fiber array in which the selective binding substances are arrayed arbitrarily and accurately with a high density may be efficiently obtained. Further, by using optical fibers, the bound sample may be detected through the fiber simply and efficiently. Further, by labeling each fiber, each fiber to which the test substance is bound may be identified without relying on the position of the fiber in the array.

Further, by the method for selective binding reaction, and the apparatus and substrate therefor, the efficiency of the selective binding reaction is promoted, so that the selective binding reaction step may be completed in a short time even when the concentration of the corresponding selective binding substance in the test sample is low.

### Brief Description of the Drawings

Fig. 1 shows a schematic cross-sectional view and plan view of the apparatus for selective binding reaction according to the present invention;
Fig. 2 is a drawing showing the principle of the behaviors of the selective binding substances in the method for selective binding reaction according to the present invention;
Fig. 3 is a schematic cross-sectional view of the conventional hybridization apparatus;
Fig. 4 is a drawing showing the principle of the behaviors of the selective binding substances in the method for selective binding reaction according to the present invention; and
Fig. 5 is a schematic view of the optical parts in the measuring apparatus used in Example 5.

### Best Mode for Carrying Out the Invention

In the present specification and claims, the term "selective binding substance" means a substance which can selectively bind to a test substance directly or indirectly. Representative examples thereof include nucleic acids, proteins, saccharides, and other antigenic compounds. The nucleic acid may be a DNA or RNA. Since a single-stranded nucleic acid having a particular nucleotide sequence selectively hybridizes with a single-stranded nucleic acid having a nucleotide sequence complementary to the nucleotide sequence of the nucleic acid or a part thereof, the single-stranded nucleic acid is a "selective binding substance" referred to in the present invention. Examples of the proteins include antibodies, antigen-binding fragments of antibodies such as Fab fragment and F(ab')₂ fragment, and various antigens. Since an antibody or a antigen-binding fragment selectively binds to the corresponding antigen, and since an antigen selectively binds to the corresponding antibody, they are "selective binding substances". As the saccharides, polysaccharides are preferred, and examples thereof include various antigens. Antigenic substances other than proteins and saccharides may also be immobilized. Especially preferred "selective binding substances" are nucleic acids, antibodies and antigens. The selective binding substance used in the present invention may be a commercially available product or one obtained from living cells or the like. The term "corresponding selective binding substance" herein means a substance which selectively binds to the above-mentioned selective binding substance. For example, in cases where the selective binding substance is a single-stranded nucleic acid, the single-stranded nucleic acid having the nucleotide sequence complementary to that of the single-stranded nucleic acid is the corresponding selective binding substance; and in cases where the selective binding substance is an antibody or an antigen-binding fragment thereof, the antigen or hapten which undergoes the antigen-antibody reaction with the antibody or an antigen-binding fragment thereof, is the corresponding selective binding substance.

Examples of the fibers which may be used for the immobilization of the selective binding substances include chemical fibers such as synthetic fibers, semi synthetic fibers, regenerated fibers and inorganic fibers, natural fibers and composite fibers of these.

Representative examples of synthetic fibers include various polyamide fibers such as nylon 6, nylon 66 and aromatic polyamides; various polyester fibers such as polyethylene terephthalate, polybutylene terephthalate, polylactic acid and polyglycolic acid fibers; various acrylic fibers such as polyacrylonitrile fibers; various polyolefin fibers such as polyethylene and polypropylene fibers; various polyvinyl alcohol fibers; various polyvinylidene fibers; polyvinyl chloride fibers; various polyurethane fibers; phenol fibers; fluorine-contained fibers such as polyfluorovinylidene and polytetrafluoroethylene fibers; and polyalkylene p-oxybenzoate fibers. Fibers other than those for clothes, for example, optical fibers mainly composed of transparent amorphous macromolecules such as polymethyl methacrylate and polystyrene may also be employed. Especially preferred are plastic optical fibers of which cores are composed of a material such as polymethyl methacrylate, polystyrene or polycarbonate, and of which clad is composed of a plastic having a smaller refractive index than the core. Either the so called sheath/core fibers or graded index fibers may be employed. Further, coated plastic optical fibers may be employed. As the coating material, thermoplastic resins such as polyethylenes, PVC, urethanes and fluorine-contained resins, and various rubber tubes may be employed.

Representative examples of semi synthetic fibers include various cellulose derivative fibers derived from diacetatc, triacetate, chitin or chitosan; and various protein-based fibers called promix. Representative examples of regenerated fibers include various cellulose-based regenerated fibers (rayon, cupra, polynosic and the like) obtained by viscose process, cuprammonium process or organic solvent process.

Representative examples of the inorganic fibers include glass fibers, carbon fibers and metal fibers such as Au, Ag, Cu and Al fibers. Especially preferred are optical fibers made of a light-transmitting glass. Similar to the plastic optical fibers, either the so called sheath/core fibers or graded index fibers may be employed. Further, coated plastic optical fibers may be employed. Glass-plastic composite fibers may also be employed.

Representative examples of natural fibers include plant fibers such as cotton, flax, ramie and jute fibers; animal fibers such as wool and silk fibers; and mineral fibers such as asbestos fibers. The form of the fiber used in the present invention is not restricted. It may be a monofilament or multifilament. Further, spun yarn obtained by spinning short fibers may also be employed. When using a fiber in the form of multifilament or yarn, the selective binding substance may be immobilized in the spaces among the filaments or the like.

The fiber used in the present invention may be a non-treated fiber, or may be one to which reactive functional groups are introduced, or may be one subjected to plasma treatment or irradiation treatment with γ-ray, electron beam or the like. Immobilization of the selective binding substance on these fibers may be attained by known methods utilizing various chemical or physical interactions between the fiber and the selective binding substance, that is, chemical or physical interactions between the functional groups on the fiber and the selective binding substance.

In cases where the same selective binding substance is immobilized, a plurality of fibers may be treated in one batch. Although selective binding substance may be immobilized on the entire fiber, it is preferred to immobilize the selective binding substance on an end region of the fiber. The end region may be the end face of the fiber and/or the side face in the vicinity of the end face. To distinguish the fibers on which different selective binding substances are immobilized, each fiber may be fixed to a support, thereby distinguishing the fibers on which different selective binding substances are immobilized. These support may be separate or connected each other. Alternatively, fibers having different shapes may be employed.

The fibers immobilizing different samples may also be distinguished by coloring the fibers and distinguishing the fibers based on the wavelengths of the colors. A method in which the light emitted to the outside from the fiber is detected, or a method in which a light passing through the fiber is guided to a photodetector, thereby distinguishing the fibers, may also be employed.

The fibers may also be distinguished by using fibers on which characters or marks are recorded. For example, bar code or the like is recorded on or in the fiber, and the fibers immobilizing different samples may be distinguished by reading the bar code or the like

Electroconductivity may be given to the fibers by incorporating an electroconductive material such as a metal, carbon, electroconductive polymer or a magnetic material in the fibers, or by coating the fibers with a material such as a metal by a method such as sputtering, vapor deposition, plating, CVD or the like. A material belonging to 3A group to 5B group in the second period to fourth period in the periodic table or a mixture thereof may be applied by the above-mentioned method. By so doing, fibers immobilizing different samples may be distinguished electrically or magnetically.

As the fibers used in the present invention, thin fibers are preferred. In the preferred mode of the present invention, the thickness of one fiber is not larger than 1 mm. As for monofilaments, commercially available fishing lines have thicknesses of 50 to 900 µm. By the recent spinning technique, a monofilament having a thickness of 1 dtex (in case polyethylene terephthalate, about 8 µm diameter) may be produced, and even thinner fiber (extra fine fiber or ultra extra thin fiber) (1 to 10 µm. diameter) may be produced. In case of using an optical fiber, the filament preferably has a diameter of 3 to 1000 µm, more preferably about 50 to 1000 µm. In view of ease of handling, plastic optical fibers, glass optical fibers and composite optical fibers made of a glass and resin, which have diameters of about 50 to 500 µm are preferred. A so called image conduit, which is a bundle of very thin optical fibers having diameters of 2 to 50 µm may also preferably be employed. The measurement may be carried out by immobilizing each selective binding substance on each image conduit, and by collecting the image conduits. In cases where the binding state is detected by light, by using this image conduit, the converged light does not diverge in the optical fiber, so that detection light may be efficiently transmitted to the sample. The number of optical fibers contained in the image conduit is preferably 2 to 50,000. If it is more than 50,000, the outer diameter of the image conduit is too large, and handling thereof may be troublesome in some cases.

In cases where an optical fiber is used, by employing a light-emitting label such as fluorescent label or a color-generating label as the label attached to the test substance hereinbelow described in detail, the binding of the test substance may be measured by measuring the light which passes through the optical fiber and emitted from another end of the optical fiber. In this case, it is also possible to identify which fiber is lighting. This is preferred, and this is advantageous for automation of the apparatus. In cases where an electroconductive fiber is used, the binding of the test substance may be measured as an electric signal at the other end of the fiber. In this case too, it is also possible to identify the fiber to which the test substance is bound, that is preferred and that is advantageous for automation of the apparatus. Further, by using an electroconductive fiber, the binding may be accelerated by using means for applying electric field or electric current. Especially, in case of accelerating the hybridization between nucleic acids, it is preferred to substantially use the fiber on which the nucleic acid is immobilized as an anode. The arrangement of a cathode is not restricted as long as the cathode does not directly contact the anode. Although the type of electric field applied is preferably direct current, alternating voltage may also be employed under a condition in which the fiber substantially acts as an anode. That is, even in cases where alternating electric field is applied, it is acceptable as long as the fiber substantially acts as an anode by making the time periods for applying positive voltage be longer than the time period for applying negative voltage, by making the absolute value of the positive voltage be larger than that of the negative voltage, or by combination of these. Although the voltage applied is not restricted, not less than 0,1V and not more than 5000V is preferred. If the voltage applied is less than 0.1 V, the effect by applying electric voltage may not be obtained sufficiently in some cases, and if it is higher than 5000V, handling may be difficult. Whether electric current flows or not when the voltage is applied is not restricted. When the cathode is arranged such that it does not contact the reaction solution, electric current does not flow, and if the cathode is arranged such that it contacts the reaction solution, electric current flows. In cases where electric current flows, 1 to 2000 mA is preferred,

On the other hand, as mentioned above, the present invention also provides a method for binding reaction between a selective binding substance and a corresponding selective binding substance, comprising immobilizing the selective binding substance on a substrate; and making a test sample contacted to the immobilized selective binding substance, which test sample contains the corresponding selective binding substance which selectively binds to the selective binding substance, thereby carrying out the binding reaction; the test sample and/or the corresponding selective binding substance being moved relative to the surface on which the selective binding substance is immobilized, in the step of immobilizing the selective binding substance on a substrate and making a test sample contacted to the immobilized selective binding substance. The phrase "moved relative to" means that the corresponding selective binding substance is moved relative to the surface immobilizing the selective binding substance when the surface immobilizing the selective binding substance is viewed laterally.

In this method for binding reaction, although it is preferred to use the above-described fiber on which the selective binding substance is immobilized or the fiber array, this is not indispensable, and the method may be applied to the methods utilizing the conventional microarrays, microplates and the like.

As the method for moving the corresponding selective binding substance in the vicinity of the selective binding substance immobilized on the microarray substrate, fiber or the like so as to increase the probability of collision between the selective binding substance and the corresponding selective binding substance, a method in which electric field is formed so as to move the electrically charged selective binding substance; a method in which a channel is formed in the substrate immobilizing the selective binding substance, and the test sample is moved using a micro pump; a method in which a function like a magnetic fluid is given to the test sample, and the test sample is moved by an external physical force; and a method in which the substrate immobilizing the selective binding substance is moved, and the like are thought.

Among these, the electric field-applying method which has an effect for increasing the probability of collision by the simplest structure, especially the method in which alternating electric field is applied is preferred. In general, nucleic acids are negatively charged in aqueous solutions. Further, macromolecules such as proteins and polysaccharides are usually also electrically charged in aqueous solutions. Therefore, by applying electric field to a reaction liquid, it is possible to move the corresponding selective binding substance. Particularly, by applying alternating electric field, it is possible to reciprocally move the corresponding selective binding substance, so that the probability of collision with the selective binding substance may be efficiently increased.

Thus, as a preferred mode for binding reaction according to the present invention, a method for binding reaction is provided wherein the binding reaction is carried out while applying an AC voltage in a direction crossing the perpendicular axis of the surface on which the selective binding substance is immobilized, between electrodes arranged outside the both ends of the area on which the selective binding substance is immobilized, in the step of immobilizing the selective binding substance on a substrate and making a test sample contacted to the immobilized selective binding substance, which test sample contains the corresponding selective binding substance which selectively binds to the selective binding substance, thereby carrying out the binding reaction.

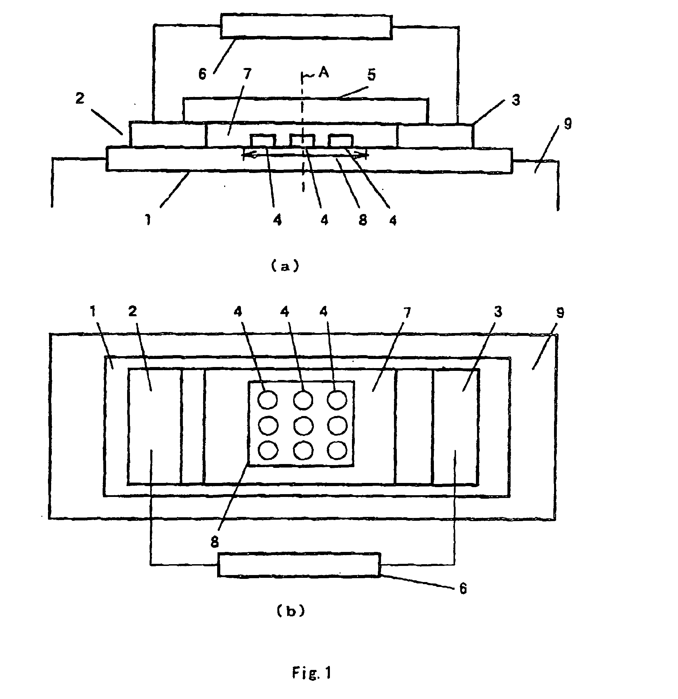

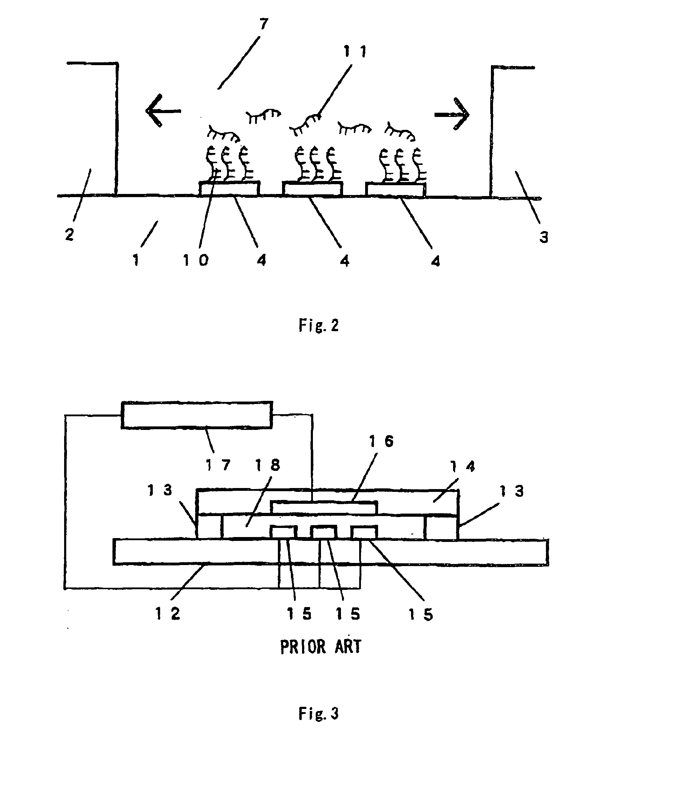

The perpendicular axis of the surface on which the selective binding substance is immobilized is, for example, the dot-and-dash line A shown in Fig. 1(a), that is, it means the direction perpendicular to the surface on which the selective binding substance is immobilized. The direction crossing this perpendicular axis means a direction which crosses the above-mentioned perpendicular axis when the surface immobilizing the selective binding substance is viewed laterally. The "direction crossing the perpendicular axis" is preferably the direction perpendicular to the perpendicular axis as shown in Fig. 2. However, the direction is not necessarily the one perpendicular to the perpendicular axis, and excellent effects may also be obtained when the angle formed by the line which connect the opposing electrodes through the shortest distance and the direction perpendicular to the perpendicular axis is not more than 45°, preferably not more than 30°.

Although the voltage applied is not restricted, if the voltage is too low, the effect obtained by applying electric field is small, and, on the other hand, if it is too high, the selective binding substance and/or the corresponding selective binding substance may be damaged. Thus, the voltage is preferably about 5V to 50V per 1 cm of the distance between the electrodes, more preferably about 10V to 25V. The frequency of the alternating voltage is not restricted, and a frequency may preferably be about 1 Hz to 100 Hz, more preferably about 5 Hz to 20 Hz.

The number of sites immobilizing the selective binding substance may be one (e.g., one well in a microplate). However, if there are a plurality of sites immobilizing selective binding substance exist, and a selective binding substance-arrayed region (e.g., the region denoted by reference numeral 8 in Fig. 1(a) mentioned below) in which the selective binding substance-immobilizing sites are arrayed exists, measurement of a plurality of types of substances may be measured simultaneously in parallel, so that it is preferred. In this case, the electrodes are preferably arranged outside the both ends of the selective binding substance-arrayed region.

The method for binding reaction by the above-described electric field-applying method may be carried out by using a substrate for conducting a binding reaction including a step of immobilizing the selective binding substance on the substrate and making a test sample contacted to the immobilized selective binding substance, which test sample contains the corresponding selective binding substance that selectively binds to the selective binding substance, thereby carrying out the binding reaction, the substrate comprising a site for immobilizing the selective binding substance; and electrodes arranged outside the both ends of the area on which the selective binding substance is immobilized. On the "site for immobilizing the selective binding substance", a selective binding substance is immobilized, and this immobilization may be carried out by an end user before use, or the manufacturer of the substrate may produce and sell one on which the selective binding substance for a particular test is immobilized beforehand.

In the method for selective binding reaction or the apparatus for selective binding reaction, examples of the materials which may be used in the electrodes include simple metals such as platinum, gold, silver, chromium, titanium, nickel, aluminum, copper and palladium, oxides and nitrides of these metals, alloys thereof, carbon and carbon compounds, and electrically conductive polymers, and it is acceptable if at least one selected from these is contained.

As for the characteristics of the simple metals, oxides and nitrides thereof, and alloys thereof, since electric current flows between the electrodes through the test sample containing corresponding selective binding substance when AC voltage is applied between the electrodes arranged using these materials, the materials which hardly react with the test sample and which hardly elute metal ions into the test sample are preferred.

Representative examples of the carbon compounds include graphite and fullerene.

Representative examples of electroconductive polymers include polyacetylene, polypyrrol, polythiophine and polyaniline. Composite electroconductive plastics having improved electroconductive properties obtained by mixing the above-mentioned electroconductive polymer and the above-mentioned metal, carbon compound or the like may also be exemplified.

Although it is preferred to form the electrodes beforehand on the substrate for immobilizing the selective binding substance because of the reason described below, the mode wherein the electrodes are arranged on the side of the apparatus for binding reaction, and wherein the electrodes are mounted on the substrate for immobilizing the selective binding substance during the step of preparation for the binding reaction is also acceptable.

Methods for forming the electrodes on the substrate using a metal include a method in which a mask with openings having the shapes of the electrodes is mounted on the substrate, and the electrodes are formed by sputtering method or vapor deposition method; a method in which electrodes in the form of thick films are formed by plating method; and a method in which metal foils or metal thin plates are adhered to the substrate by an adhesive. In case of using a carbon compound, the electrodes may be prepared by mounting a mask with openings having the shapes of the electrodes, and forming the electrodes by sputtering method. In case of using an electroconductive polymer, the electrodes may be formed by applying an electroconductive polymer in the form of paste by a printing method such as silk screen printing, and by curing the paste by photo-curing by UV light.

In cases where the electrode are retained in the apparatus for selective binding reaction, electrode plates in the form of a thin plate prepared by using the above-mentioned metal material, carbon compound or the electroconductive polymer are arranged on the base of the apparatus for selective binding reaction, and the electrode plates are mounted on the substrate for immobilizing the selective binding substance after mounting the substrate for immobilizing the selective binding substance on the base, thereby forming the electrodes.

A mode of the present invention will now be described referring to the drawings. The side view and the plan view of a mode of the apparatus for selective binding reaction according to the present invention are shown in Fig. 1 (a) and Fig. 1(b), respectively. It should be noted that the present invention is not limited to this example. As shown in Figs. I and 2, the apparatus comprises a substrate 1 on which selective binding substances are arrayed, electrodes 2 and 3, a cover plate 5 and means for applying AC voltage 6. Selective binding substances 10 are immobilized on the sites 4 for immobilizing selective binding substance arranged on the selective binding substance-arraying substrate 1, thereby forming a selective binding substance-arrayed region 8. On the selective binding substance-arraying substrate 1 mounted on a base 9, electrodes 2 and 3 are arranged on both sides of the selective binding substance-arrayed region 8, and a cover plate 5 bridging the electrodes 2 and 3 is mounted on the electrodes 2 and 3. The space sandwiched between the selective binding substance-arraying substrate 1 and the cover plate 5 via electrodes 2 and 3 is filled with a test sample solution 7 containing corresponding selective binding substances 11.

Although it is preferred to array the selective binding substances 10 on the lattice points of a two-dimensional lattice in the selective binding substance-arrayed region 8, they may be arranged on the positions shifted from the lattice points, or may be arranged along a straight line. Alternatively, the sites 4 for immobilizing selective binding substance may be arranged three-dimensionally in the direction perpendicular to the surface of the selective binding substance-arraying substrate 1 such that they form steps.

After filling the above-mentioned space with the test sample solution 7, a voltage is applied between the electrodes 2 and 3 using the means for applying AC voltage 6. By this, an electric field is generated between the electrodes 2 and 3. Since the corresponding selective binding substances 11 naturally diffused in the test sample solution 7 have negative charges, they repeat movement in the direction crossing the selective binding substance-arrayed region 8 in accordance with the direction of the electric field generated between the electrodes 2 and 3. More particularly, when the electrode 2 is positively biased and electrode 3 is negatively biased, the corresponding selective binding substances 11 are attracted to the electrode 2, and when the electrode 2 is negatively biased and electrode 3 is positively biased, the corresponding selective binding substances 11 are attracted to the electrode 3. During the movement of the corresponding selective binding substances crossing the selective binding substance-arrayed region 8, the corresponding selective binding substances 11 contact the selective binding substances 10 immobilized on the sites 4 for immobilizing the selective binding substance, so as to attain hybridization when they have complementary sequences.

The higher the voltage applied between the electrodes, the larger the electric attracting force and the electric repulsive force exerted to the corresponding selective binding substances 11 by the electrodes, and so the higher the effects of the contact of the corresponding selective binding substances 11 with the selective binding substances 7 due to the movement of the corresponding selective binding substances 11. However, if a high voltage is applied for a long time, the selective binding substances 7 and the corresponding selective binding substances 11 may be damaged. Therefore, in this mode, the voltage per 1 cm of the distance between the electrodes is 5V to 50V, and a voltage of 10V to 25V per 1 cm of the distance between the electrodes is more preferred in order to attain stable hybridization.

As explained above, in the method for promoting efficiency of selective binding reaction by using AC electric field according to the present invention, the selective binding substances 10 and the corresponding selective binding substances 11 are always moved relatively during the binding reaction, and so repeat collision and contact therebetween, the efficiency of the binding reaction is promoted.

As the sites for immobilizing the selective binding substance, areas in a surface on the substrate or a part of the substrate, hollow or protruded regions formed on the substrate are usually employed. Alternatively, rod-shaped resin, glass, metal, fibers or the like are inserted into holes penetrating the selective binding substance-arraying substrate 1, and the ends of the resin, glass, metal and fibers may be used as the sites for immobilizing the selective binding substance. Especially, by using the end faces of the above-described fibers or fiber bundle according to the present invention as the sites for immobilizing the selective binding substance, detection at the other end of the each of the fibers may be attained, so that it is preferred.

To reduce the amount of the valuable test sample solution 7, the thickness of the electrodes 2 and 3 is preferably as small as possible, more preferably 5 µm to 200 µm. To form such very thin electrodes of which irregularities in the thickness are small, it is preferred to form the electrodes on the selective binding substance-arraying substrate beforehand. However, it is also acceptable to provide the electrodes on the side of the apparatus for binding reaction, and mounting the electrodes on the selective binding substance-arraying substrate 1 during the step of preparation for the binding reaction.

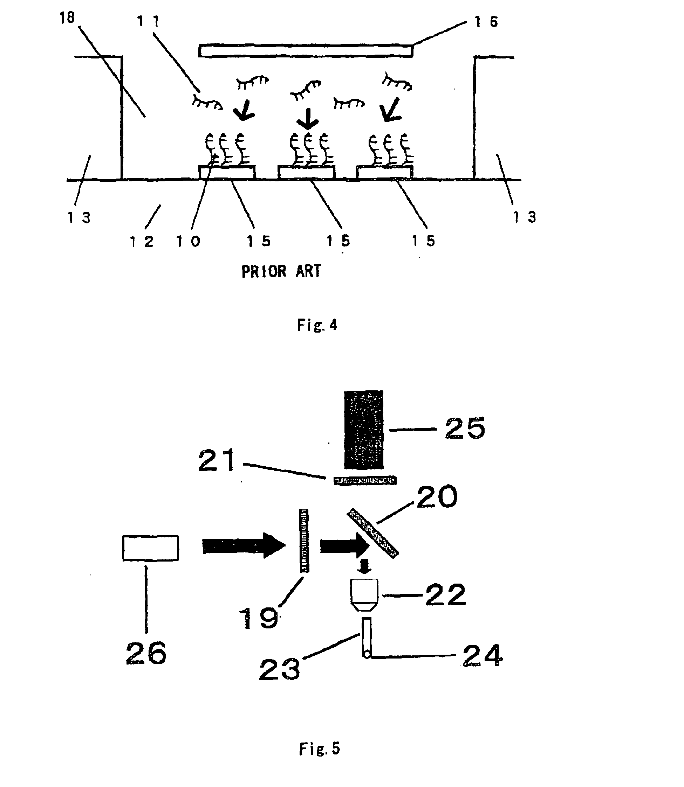

In the conventional methods, since the hybridization reaction depends on the natural diffusion of the selective binding substances, the probability of contact between the selective binding substances 10 and the corresponding selective binding substances 11 is low, so that the efficiency of hybridization reaction is low. Further, in the methods which tried to overcome this inefficiency by utilizing electric attraction as shown in Figs. 3 and 4, the promotion of the efficiency is not sufficient.

The conventional methods for promoting hybridization efficiency utilizing electric attraction will be described referring to Figs. 3 and 4. The selective binding substances 10 are immobilized on sites 15 for immobilizing selective binding substance formed on a selective binding substance-arraying substrate 12. On the selective binding substance-arraying substrate 12 and outside the region in which the selective binding substances 10 are arrayed, supports 13 are arranged, and a cover plate 14 bridging the supports 13 is mounted. The space sandwiched between the selective binding substance-arraying substrate 12 and the cover plate 14 via the supports 13 is filled with a test sample solution 18. After filling the space with the test sample solution 18, an electrode 16 is negatively biased, and the sites 15 for immobilizing selective binding substance having an electroconductive layer is positively biased using means for applying electric voltage 17. As a result, an electric field is generated between the electrode 16 and the sites 15 for immobilizing selective binding substance, Since the corresponding selective binding substances 11 naturally diffused in the test sample solution 18 have negative charges, they are attracted to the sites 15 for immobilizing selective binding substance. As a result, the concentrations of the corresponding selective binding substances 11 in the vicinity of the sites 15 for immobilizing selective binding substance are increased, so that the selective binding substance 10 and the corresponding selective binding substance 11 contact during the process of adsorption of the corresponding selective binding substances 11.

However, by the method shown in Fig, 3, the corresponding selective binding substances 11 are adsorbed on the sites 15 for immobilizing selective binding substance by the electric force, and the selective binding substances 10 having negative charges similar to the corresponding selective binding substances 11 are also adsorbed on the surfaces of the selective binding substance-immobilizing sites 15, so that the relative movements between the selective binding substance 10 and the corresponding selective binding substance 11 become small. Therefore, the probability of contact between the selective binding substances 10 and the corresponding selective binding substance 11 is smaller than in the method according to the present invention.

The DNA or RNA used as the selective binding substance or corresponding selective binding substance may be those prepared from living cells or may be those chemically synthesized. Preparation of the DNA or RNA from living cells may be carried out by known methods. For example, extraction of DNA may be carried out by the method of Blin et al., (( Blin et al., Nucleic Acids Res. 3: 2303 (1976)), and extraction of RNA may be carried out by the method of Favaloro et al., (Favaloro et al., Methods Enzymol.65: 718 (1980)). The nucleic acids to be immobilized may also be lincar or circular plasmid DNAs and chromosomal DNAs, DNA fragments obtained by cleaving these DNAs by a restriction enzyme or obtained by chemically cleaving these DNAs, synthetic DNAs prepared *in vitro* by using an enzyme or the like, chemically synthesized oligonucleotides or the like.

On one fiber or on each site for immobilizing selective binding substance, one type of selective binding substance is usually immobilized. However, a plurality of types of selective binding substances may be immobilized on one fiber or one site for immobilizing selective binding substance in cases, for example, where a plurality of types of genes having mutations are desired to be immobilized on the same site for immobilization.

As the selective binding substance immobilized on a plurality of fibers or on a plurality of sites for immobilizing selective binding substance, different types of selective binding substances may be immobilized on the different fibers or the different sites, or the same type of selective binding substance may be immobilized on the different fibers or the different sites. Alternatively, one type of selective binding substance may be immobilized on a part of the fibers or a part of the sites for immobilizing selective binding substance, and another type of selective binding substance may be immobilized on a plurality of other fibers or other sites for immobilizing selective binding substance. The types of the selective binding substances and order of arrangement are not limited by the positions of the fibers in the fiber array. It is also effective to immobilize the same selective binding substance on a plurality of fibers or a plurality of sites for immobilizing selective binding substance, thereby increasing the measurement sensitivity.

Immobilization of the selective binding substance on the support fiber or on the site for immobilizing selective binding substance may be carried out by a known method. In cases where an unmodified selective binding substance is immobilized on the fiber or on the site for immobilizing selective binding substance, the selective binding substance may be immobilized by making the selective binding substance contact the fiber or the site for immobilizing selective binding substance, and then subjecting the resultant to baking or UV irradiation. In the Examples below, DNAs are immobilized on polymethyl methacrylate optical fibers or polymethyl methacrylate substrate by this method. In cases where a selective binding substance modified with amino groups is to be immobilized on a fiber, the selective binding substance may be bound to a functional group in the fiber using a crosslinking agent such as glutaraldehyde or 1-ethyl-3-(3-dimethylaminopropyl)carbodiimide (EDC). The temperature at which the sample containing the selective binding substance is made to contact the fiber is preferably 5°C to 95°C, more preferably 15°C to 65°C. The treatment time is usually 5 minutes to 24 hours, and preferably not less than 1 hour.

In the present invention, the selective binding substance may be immobilized on the fiber or the site for immobilizing selective binding substance as it is. Alternatively, a derivative of the selective binding substance obtained by chemical modification, or a denatured nucleic acid may be immobilized as required. Known chemical modification of nucleic acids include amination, biotinylation and attachment of digoxigenin [Current Protocols In Molecular Biology, Ed.; Frederick M. Ausubel et al.(1990); and (Non-Isotope Experimental Protocol (1) DIG Hybridization (Shujunsha)], and these modification methods may be employed in the present invention. As an example, introduction of amino groups into a nucleic acid will now be described. The positions at which the aliphatic hydrocarbon chain having an amino group and the nucleic acid are bound are not restricted, and may be not only the 5'-end or 3'-end of the nucleic acid, but also may be within the nucleic acid chain (e.g., phosphoric acid diester moiety or base moiety). This single-stranded nucleic acid derivative may be prepared in accordance with methods described in Japanese Laid-open Patent Application (Kokai) No. 3-74239, U.S. Patent No. 4,667,025 and U.S. Patent No. 4,789,737. Other than this method, the amino group may be introduced by, for example, using a commercially available reagent for introducing amino groups [e.g., Aminolink II (trademark), PE BIOSYSTEMS JAPAN, or Amino Modifiers (trademark), CLONETECH], or by a well-known method (Nucleic Acids Res., 11(18),6513-(1983)) for introducing amino group into the phosphate at the 5'-end of the DNA.

The fiber immobilizing the selective binding substance or the substrate immobilizing the selective binding substance may be subjected to an appropriate treatment after immobilizing the selective binding substance. For example, the immobilized selective binding substance may be denatured by heat treatment or by treatment with an alkali or a surfactant. In case of using a selective binding substance obtained from a biological material such as a cell or bacterium, unnecessary cellular components may be removed- The fiber or the substrate for immobilizing selective binding substance after the treatment may be used as a material for detecting the selective binding substance. These treatments may be performed separately or simultaneously. Alternatively, the treatment may be performed before a sample containing the selective binding substance is immobilized on the fiber or the substrate for immobilizing selective binding substance.

By making the fiber or the substrate for immobilizing selective binding substance according to the present invention on which the selective binding substance is immobilized as a probe interact with a test substance, the particular test substance in the sample may be detected. Labeling (so as to attain distinguishment) described below may be performed on two types of test samples, and the difference may be compared.

For the detection of the corresponding selective binding substance in the test sample, which selectively binds to the selective binding substance, known means for specifically recognizing the binding may be employed. For example, a label such as a fluorescent substance, luminescent substance, radio isotope is bound to the corresponding selective binding substance in the sample, and after the selective binding reaction and washing, the label may be detected. The type of the label and the method for introducing the label are not restricted at all, and various known means may be employed. Particularly, in cases where the fiber transmits light, and the labeled test substance is subjected to the reaction at an end of the fiber, and the label is detected with a detector at the other end of the fiber, it is preferred to use a fluorescent substance or luminescent substance as the label. Fluorescent substances and luminescent substances used for immunoassays and for the measurements of hybridization of nucleic acids are well-known in the art and various such substances are commercially available. These commercially available fluorescent substances and luminescent substances may be employed.

The measurement of the result of the binding reaction may also be carried out by reacting a free labeled substance for measurement, which selectively binds to the corresponding selective binding substance after or simultaneously with the binding reaction between the selective binding substance immobilized on the fiber or on the site for immobilizing the selective binding substance and the corresponding selective binding substance in the sample, and by measuring the label of the substance for measurement immobilized on the fiber via the corresponding selective binding substance and the selective binding substance after washing. For example, in a case where a nucleic acid having a particular nucleotide sequence as a selective binding substance is immobilized on a fiber, and the corresponding selective binding substance is a nucleic acid containing a region complementary to the nucleic acid, a nucleic acid complementary to a region in the nucleic acid which is the corresponding selective binding substance, other than the region complementary to the selective binding substance, may be used as the substance for measurement after labeling. Similarly, in a case where an antigen as the selective binding substance is immobilized on a fiber, and the corresponding selective binding substance is an antibody which undergoes antigen-antibody reaction with the antigen, a labeled secondary antibody which undergoes antigen-antibody reaction with the antibody may be used as the substance for measurement. In these cases, it is preferred to immobilize the selective binding substance on one end region of the fiber, to employ a fluorescent or luminescent substance as the label, and to measure the result of the reaction from the other end of the fiber.

In cases where the fiber or the site for immobilizing selective binding substance is electrically conductive, it is preferred to employ a method for detecting a response such as electric current based on an electrochemical reaction. In this case, existence or degree of binding between the selective binding substance and the test sample may be detected by reacting the selective binding substance immobilized on the fiber acting as an electrode and the test sample in the presence of a material which accelerates or suppresses the reaction between the selective binding substance and the test sample, wherein all or a part of the material is contained in the bound selective binding substance and test sample, and by measuring the electric current flowing the electrode, that is, the fiber or the site for immobilizing the selective binding substance after the reaction.

Examples of the test substances subjected to the measuring method using the fiber or fiber array according to the present invention, or to a measuring method using the substrate for immobilizing selective binding substance applied to the apparatus for binding reaction according to the present invention include genes of pathogenic bacteria, viruses and the like, causative genes of hereditary diseases and. parts thereof, various biological components having antigenecities, and antibodies to pathogenic bacteria, viruses and the like, although the test substances are not restricted thereto. Examples of the samples containing such test substances include body fluids such as blood, serum, plasma, urine, feces, spinal fluid, saliva and various tissue fluids; various foods and beverages as well as dilutions thereof. The nucleic acid which is a test substance may be a labeled nucleic acid extracted from blood or cells by a conventional method and labeled, or may be an amplified nucleic acid obtained by a nucleic acid-amplification method such as PCR. In the latter case, the measurement sensitivity may be largely promoted. In cases where an amplification product of a gene is used as the test substance, by carrying out the amplification in the presence of a nucleoside triphosphate labeled with a fluorescent substance or the like, the amplified nucleic acid can be labeled. In cases where the test substance is an antigen or an antibody, the antigen or antibody which is the test substance may be directly labeled by a conventional method. Alternatively, after binding the antigen or antibody which is the test substance with the selective binding substance, the fiber or the site for immobilizing selective binding substance on which the selective binding substance has been immobilized is washed, reacting a labeled antibody or antigen which undergoes antigen-antibody reaction with the antigen or antibody, and the amount of the label bound to the fiber or to the site for immobilizing selective binding substance is measured.

The step of interacting the immobilized substance and the test substance may be carried out in exactly the same manner as in the conventional method. The reaction temperature and the time are appropriately selected depending on the length of the chain of the nucleic acids to be hybridized, type of the antigen and/or antibody involved in the immune reaction. Usually, in case of hybridization of nucleic acids, the reaction may be carried out at about 50°C to 70°C for 1 minute to several hours, and in case of immune reaction, the reaction may be carried out at room temperature to about 40°C for 1 minute to several hours.

By the above-described method, the amount of the test substance such as nucleic acid, antibody or antigen, which selectively binds to the immobilized selective binding substance may be measured. That is, in cases where a nucleic acid is immobilized as the selective binding substance, the amount of a nucleic acid having a sequence complementary to the immobilized nucleic acid or a part thereof may be measured. In cases where an antibody or antigen is immobilized as the selective binding substance, the amount of the antigen or antibody which undergoes immune reaction with the immobilized antibody or antigen may be measured. It should be noted that the term "measure" used in the present specification include both detection and quantification.

By using the present invention, expression of genes, proteins and sugar chains in various organisms may be investigated efficiently, quickly and simply. For example, after labeling the nucleic acids extracted from a normal human liver and a liver infected with a hepatitis virus, the nucleic acids are subjected to hybridization with each of the various known human genes immobilized on the fibers or on the substrate for immobilizing selective binding substance according to the present invention. By comparing the degree of binding of the nucleic acids extracted from the normal and hepatitis-infected livers, the change in the gene expression in the hepatitis liver may be investigated.

Similarly, by binding the proteins extracted from a normal brain and from a brain suffering from Alzheimer's disease are subjected to a binding reaction with a fiber array on which various monoclonal antibodies which are proteins are immobilized, and by comparing the bound proteins between the normal and Alzheimer's brains, abnormal expression of proteins in Alzheimer's brain may be investigated.

### Examples

The present invention will now be described in more detail by way of examples below. However, the present invention is not limited to the examples below.

### Example 1

To confirm that immobilization of nucleic acids on the fiber or glass substrate used in this example and hybridization thereof can be carried out surely, experiments were carried out using digoxigenin as a label, which does not cross-react with biological samples, and which is thermally stable and is not decomposed by the heat given when the hybridization is carried out.

One end of each of polymethyl methacrylate optical fibers was dipped in a nucleic acid solution of actin gene (produced by Takara Shuzo Co., Ltd.) (concentration of the nucleic acid: 10 µg/ml), and the resultant was subjected to UV treatment (UV Crosslinker produced by Stratagene) after drying in the air, to obtain fibers on which the nucleic acid was immobilized. An oligonucleotide having a nucleotide sequence complementary to a part of the sequence of the used nucleic acid was synthesized, and labeled with digoxigenin (DIG, Roche Diagnostics).

On the other hand, the nucleic acid solution of actin gene (produced by Takara Shuzo Co., Ltd.) (concentration of the nucleic acid: 10 µg/ml) was spotted on a slide glass to which amino groups had been introduced, such that each size of the immobilized site had a diameter of about 200 µm, and the resultant was subjected to UV treatment (UV Crosslinker produced by Stratagene) after drying in the air, to obtain a substrate on which the nucleic acid was immobilized. The oligonucleotide having a nucleotide sequence complementary to a part of the sequence of the used nucleic acid was synthesized, and labeled with digoxigenin (DIG, Roche Diagnostics).

Each oligonucleotide of which end was aminated was dissolved in 100 mM boric acid buffer (pH8-5) to a final concentration of 2 mM. Equal amount of digoxigenin-3-O-methylcarbonyl-a-aminocaproic acid-N-hydroxy-succinimide ester (26 mg/ml solution in dimethylformamide) was added to the solution, and the resulting mixture was left to stand overnight at room temperature, Ethanol precipitation was performed using glycogen (Roche Diagnostics) as a carrier, and the precipitates were dissolved in 100 µmol of 10 mM Tris-HCl (pH7.5) and 1 mM EDTA after drying in the air. The thus obtained DIG-labeled oligonucleotide was used as a model of the sample nucleic acid.

The prepared nucleic acid-immobilized fibers were placed in a bag for hybridization, and hybridization was carried out by a conventional method (in accordance with the instructions in the manual of the product of Roche Diagnostics).

On the other hand, the prepared nucleic acid-immobilized substrate was mounted on a base of an apparatus for hybridization, and hybridization was carried out by a conventional method (in accordance with the instructions in the manual of the product of Roche Diagnostics).

After completion of the hybridization, the nucleic acid-immobilized fibers and the nucleic acid-immobilized substrate were washed, and an enzyme-labeled anti-DIG antibody solution was added to allow antigen-antibody reaction to occur. After the reaction, the nucleic acid-immobilized fibers and the nucleic acid-immobilized substrates were washed to remove the unbound antibody. A DIG-detecting reagent was added and the mixture was equilibrated. After draining water, optical signals were detected. As a result, signals were detected depending on the immobilization of the nucleic acid.

By this, it was confirmed that the apparatus for hybridization according to the present invention may be used surely as an apparatus for hybridization in the conventional method not applying an AC voltage without a constitutional or functional problem

### Example 2

### Pretreatment of Optical Fibers and Substrate for Immobilizing Selective Binding Substance

Each glass optical fiber having a diameter of 200 µm (produced by Hoya Shot) was cut with a special cutter for optical fibers, and both end faces of the fiber were mirror-finished.

The thus processed optical fibers and a slide glass (76mm x 26mm x 1 mm) (produced by Matsunami Glass Kogyo) were cleaned with a mixed solution of pure water, ethanol and NaOH, and then washed with pure water. The cleaned surface was immersed in a mixed solution of pure water and poly-L-lysine (composition: 10% poly-L-lysine) to introduce amino groups to the surface of the slide glass.

Using two types of nucleic acid solutions ("λControl Template & Primer SetA" produced by Takara Shuzo Co., Ltd.; Product No. TX803 (λDNA fragment of about 1000 bp) and "Human TFR(1kb) Template & Primer Set" produced by Takara Shuzo Co., Ltd.; Product No. TX806 (DNA fragment of human transferrin receptor of about 1000 bp), each of the nucleic acids was amplified by PCR method. The primers used in the PCR method were those contained in each commercial product. The amplification products were purified to obtain purified nucleic acid solutions. The two types of nucleic acid solutions were spotted on the surface of the slide glass, to which amino groups were introduced, and the resultant was subjected to UV crosslinking (120 mJ) after drying in the air to obtain a nucleic acid-immobilized substrate in which two types of nucleic acids were immobilized on the sites for immobilizing nucleic acids. To block the remaining amino groups on the slide glass surface, which did not react with the nucleic acids, the surface on which the nucleic acids were immobilized was immersed in a mixed solution of boric acid, pure water, NaOH for adjusting pH, succinic anhydride and 1-methyl-2-pyrrolidone (prepared by dissolving 3 g of succinic anhydride in 187 ml of 1-methyl-2-pyrrolidone, and 17 ml of 1M Na-borate (pH8.0) was added immediately before use) and shaken, followed by washing the surface.

### Treatment of RNA

An RNA solution ("λpolyA+RNA-A) produced by Takara Shuzo Co., Ltd.; Product No. TX802) was provided. This RNA has a nucleotide sequence complementary to that of one of the above-described nucleic acids (TX803). The RNA solution was mixed with reverse transcriptase (Super script II, produced by GIBCO BRL; Product No. 18064-071), 2.5 mM dATP, 2.5 mM dCTP. 2.5 mM dGTP, 1.0 mM dTTP, Cy5-dUTP (produced by Amersham Pharmacia; Product No. PA55022), and the resulting mixture was incubated at 42°C for 1 hour, to obtain a cDNA solution to which Cy5 dye was incorporated.

Similarly, an RNA solution ("Human TFR RNA (1 kb)" produced by Takara Shuzo Co., Ltd.; Product No. TX805) was provided. The step of reverse transcription was carried out in the same manner as described above except that Cy3-dUTP (produced by Amersham Pharmacia; Product No. PA53022) was used in place of Cy5-dUTP to obtain a cDNA solution in which Cy3 dye was incorporated. This cDNA in which Cy3 dye was incorporated has a nucleotide sequence complementary to that of one of the above-described nucleic acids (TX806).

The two types of cDNA solutions to which the above-described dyes were incorporated were mixed, purified and dissolved in a buffer (3.4 x SSC, 0.1% SDS) to obtain a solution for hybridization.

### Hybridization

Two optical fibers each in which one type of nucleic acid was immobilized on one end face thereof and the cDNA solution (solution for hybridization) containing the above-described dyes were placed in a vinyl bag, and the bag was tightly closed such that the solution for hybridization is not evaporated. The resultant was left to stand at 65°C for 16 hours. The optical fibers were then taken out of the vinyl bag and washed.

### Detection of Fluorescence

Detection of fluorescence from Cy5 using the optical fiber was carried out as follows. As the excitation light of the fluorescence, laser (wavelength 635 nm) was used. The condensed laser beam was irradiated on the end face of the optical fiber immersed in the DNA solution, and the light emitted from another end face was condensed a with condenser lens, A dichroic mirror (produced by Omega Optical; Product No. XF2035) was arranged downstream thereof at an angle of 45° to the optical axis, thereby removing extra excitation light. Immediately downstream of the dichroic mirror, a bandpass filter (produced by Omega Optical; Product No. XF3076) was arranged to further remove extra excitation light. Immediately downstream of the bandpass filter, Photomultimeter (produced by Hamamatsu Photonics; Product No. H5784-02) was arranged and fluorescence from Cy5 dye was observed.

To measure the fluorescence from Cy5 using the nucleic acid-immobilized substrate, an optical system was prepared as follows. *As* the excitation light of the fluorescence, laser (wavelength 635 nm) was used. A bandpass filter (produced by Omega Optical; Product No. X1069) was arranged perpendicularly to the optical axis to remove extra light except for the excitation light A dichroic mirror (produced by Omega Optical; Product No. XF2035) was arranged at an angle of 45° to the optical axis of the laser beam, and the condensed beam was irradiated on the end surface of the optical fiber, which end surface is opposite to the end surface immersed in the DNA solution. Further, the fluorescence returned from an end surface immersed in the DNA solution was condensed at the side of the end surface on which the excitation light was irradiated, and the fluorescence was made to pass through the above-mentioned dichroic mirror (produced by Omega Optical; Product No. XF2035) and then the bandpass filter (produced by Omega Optical; Product No, X1069) to cut extra excitation light.

In both cases using the optical fiber and the nucleic acid-immobilized substrate, the fluorescence from Cy3 was detected in the same manner as described above except that the dichroic mirror and the bandpass filters were replaced with those for Cy3 (produced by Omega Optical; Product Nos. XF1074, XF2017 and XF3083, respectively) and that the wavelength of the irradiated laser was 532 nm.

By these methods, the fluorescences from the two types of optical fibers and from the nucleic acid-immobilized sites were detected for *Cy5* and Cy3. From the optical fiber prepared by immersing the fiber in the nucleic acid solution of TX803, fluorescence from Cy5 alone was observed, and fluorescence from Cy3 was not observed. From the optical fiber prepared by immersing the fiber in the nucleic acid solution of TX806 and from the nucleic acid-immobilized site prepared by spotting of the nucleic acid solution of TX806, fluorescence from Cy3 alone was observed, and fluorescence from Cy5 was not detected.

### Example 3

The same procedures as in Example 2 were repeated except that the optical fibers prepared were bundled, the beam of the excitation light was scanned, and that fluorescence from each optical fiber was detected. As a result, the same results as in Example 2 were obtained.

### Example 4

The same procedures as in Example 2 were repeated except that the end face of the optical fiber, which is irradiated with the excitation laser was the end face not immersed in the DNA solution. That is, the procedures were the same as in Example 2 except that the direction of the optical fibers when detecting the fluorescence was opposite to that employed in Example 2. As a result, the same results as in Example 2 were obtained.

### Example 5

Treatments of optical fibers and nucleic acids were carried out in the same manner as in Example 2.

To measure the fluorescence from Cy5, the optical system was constructed as follows (see Fig. 5). As the excitation light of the fluorescence, laser 26 (wavelength 635 nm) was used. A bandpass filter 19 (produced by Omega Optical; Product No. X1069) was arranged perpendicularly to the optical axis to remove extra light except for the excitation light. A dichroic mirror 20 (produced by Omega Optical; Product No. XF2035) was arranged at an angle of 45° to the optical axis of *the* laser beam, and the condensed beam was irradiated on the surface of the slide glass opposite to the surface immersed in the DNA solution. Further, the fluorescence returned from an end surface immersed in the DNA solution was condensed at the side of the end surface on which the excitation light was irradiated, and the fluorescence was made to pass through the above-mentioned dichroic mirror 20 (produced by Omega Optical; Product No. XF2035) and then the bandpass filter 21 (produced by Omega Optical; Product No. X3076) to cut extra excitation light. In Fig. 5, reference numeral 22 denotes the condenser lens, 23 denotes the optical fiber, 24 denotes a sample (DNA) and 25 denotes a photomultiplier.

The fluorescence from Cy3 was detected in the same manner as described above except that the dichroic mirror and the bandpass filters were replaced with those for Cy3 (produced by Omega Optical; Product Nos. XF1074, XF2017 and XF3083, respectively) and that the wavelength of the irradiated laser was 532 nm.

By these methods, the fluorescence from the two types of optical fibers was measured for Cy5 and Cy3. From the optical fiber prepared by immersing the fiber in the nucleic acid solution of TX803, fluorescence from Cy5 alone was observed, and fluorescence from Cy3 was not observed. From the optical fiber prepared by immersing the fiber in the nucleic acid solution of TX806, fluorescence from Cy3 alone was observed, and fluorescence from Cy5 was not detected.

### Example 6

As the optical fibers, image conduits (Produced by Edmont Optics Japan; Product No. CJ53843) constituted by bundling a number of thin optical fibers were used. This image conduit was formed of 3012 quartz optical fibers each of which had a thickness of 25 µm.

The treatments of the optical fibers and the nucleic acids were carried out in the same manner as in Example 5. The detection optical system was the same as in Example 5. As a result, from the optical fiber prepared by immersing the fiber in the nucleic acid solution of TX803, fluorescence from Cy5 alone was observed, and fluorescence from Cy3 was not observed. From the optical fiber prepared by immersing the fiber in the nucleic acid solution of TX806, fluorescence from Cy3 alone was observed, and fluorescence from Cy5 was not detected. Taking the fluorescence intensities from Cy5 and Cy3 in Example 5 are taken as 1, the fluorescence intensities in this example were 3, so that the S/N ratio was increased. The reason for this is thought to be that the condensed light does not diverge in the optical fiber, and the excitation light can be efficiently transmitted to the sample, when an image conduit is used.

### Example 7

### Treatment of Optical Fiber

The glass optical fibers (produced by Hoya Shot) having a diameter of 200 µm used in Example 1 were coated with Pt film by sputtering to obtain electroconductive optical fibers. The glass optical fibers were cut with a special cutter for optical fibers, and both end faces of the fibers were mirror-finished. The end face of each optical fiber was immersed in a mixed solution of pure water and poly-L-lysinc (composition: 10% poly-L-lysine) to introduce amino groups on one end face of each optical fiber.

The treatment of the nucleic acids and so on thereafter were the same as in Example 2, to obtain two optical fibers each in which one type of nucleic acid was immobilized on one end face thereof, and the solution for hybridization.

### Hybridization

The two optical fibers each in which one type of nucleic acid was immobilized on one end face thereof, and the cDNA solution (solution for hybridization) to which the dyes Cy5 and Cy3 were incorporated were placed in a microtube (1.5 ml). A hole was formed in the cap of the microtube, and the optical fibers were passed through the hole. The end face of each optical *fiber,* on which the DNA was immobilized was immersed in the solution for hybridization in the microtube. The hole in the cap of the microtube was sealed with paper bond such that the solution is not evaporated. The volume of the solution for hybridization was 50 µl. The solution was one in which the labeled DNA was dissolved in pure water. The resultant was left to stand at 65°C for 10 minutes. During this, a voltage of 100V was applied such that the optical fibers were used as the anode and a copper foil attached to the bottom of the microtube was used as the cathode. The optical fibers were taken out of the microtube and washed. The light was measured in the same manner as in Example 2. As a result, from the optical fiber prepared by immersing the fiber in the nucleic acid solution of TX803, fluorescence from Cy5 alone was observed, and fluorescence from Cy3 was not observed. From the optical fiber prepared by immersing the fiber in the nucleic acid solution of TX806, fluorescence from Cy3 alone was observed, and fluorescence from Cy5 was not detected. The fluorescence intensities were the same as those in Example 2. On the other hand, when the treatment conditions of the optical fibers were the same as in Example 2 (i.e., electric voltage is not applied), and the hybridization time was 10 minutes, the fluorescence intensities were 1/3 of those in Example 2. Thus, it was proved that by making the optical fiber electroconductive, and by applying electric field, even a period of only 10 minutes is sufficient as the hybridization time.

### Example 8

Pretreatment of Substrate for Immobilizing Selective Binding Substance

To form electrodes on the selective binding substance-arraying substrate, a stainless mask having two openings each of which sized 10 mm x 5 mm and which were arranged such that the side of the 10 mm of each opening faces each other in parallel via an interval of 10 mm was mounted on a slide glass, and gold electrodes corresponding to the openings of the mask were formed on the slide glass by sputtering method,

Thus prepared slide glass (76 mm x 26 mm x 1 mm) (produced by Matsunami Glass Kogyo) on which the gold electrodes were arranged was cleaned with a mixed solution of pure water, ethanol and NaOH, and then washed with pure water. The cleaned surface was immersed in a mixed solution of pure water and poly-L-lysine (composition: 10% poly-L-lysine) to introduce amino groups to the surface of the slide glass.

Using two types of nucleic acid solutions ("λControl Template & Primer SetA" produced by Takara Shuzo Co., Ltd.; Product No. TX803 (λDNA fragment of about 1000 bp) and "Human TFR(1kb) Template & Primer Set" produced by Takara Shuzo Co., Ltd.; Product No. TX806 (DNA fragment of human transferrin receptor of about 1000 bp), each of the nucleic acids was amplified by PCR method. The primers used in the PCR method were those contained in each commercial product. The amplification products were purified to obtain purified nucleic acid solutions. The two types of nucleic acid solutions were spotted on the surface of the slide glass between the gold electrodes, to which amino groups were introduced, and the resultant was subjected to UV crosslinking (120 mJ) after drying in the air to obtain a nucleic acid-immobilized substrate in which two types of nucleic acids were immobilized on the sites for immobilizing nucleic acids. To block the remaining amino groups on the slide glass surface, which did not react with the nucleic acids, the surface on which the nucleic acids were immobilized was immersed in a mixed solution of boric acid, pure water, NaOH for adjusting pH, succinic anhydride and 1-methyl-2-pyrrolidone (prepared by dissolving 3 g of succinic anhydride in 187 ml of 1 -methyl-2-pyrrolidone, and 17 ml of 1M Na-borate (pH8.0) was added immediately before use) and shaken, followed by washing the surface.

### Treatment of RNA

An RNA solution ("λpolyA+RNA-A) produced by Takara Shuzo Co., Ltd.; Product No. TX802) was provided. The RNA solution was processed in the same manner as in Example 2 to obtain a cDNA solution in which Cy5 dye was incorporated, and a solution for hybridization.

### Hybridization.

The substrate for immobilizing nucleic acid, on which surface the two types of nucleic acids were immobilized was fixed on a base of the hybridization apparatus, and the gold electrodes arranged on both sides of the nucleic acid-immobilized sites were connected to means for applying AC voltage. On the sites on which the two types of the nucleic acids were immobilized, 2 µl of the above-described solution for hybridization was dropped, and a cover plate bridging the electrodes formed on both sides of the nucleic acid-immobilized sites was placed. The cover plate was closed tightly so as not to allow evaporation of the solution for hybridization. An AC voltage of 10V, 10Hz was applied between the electrodes and the solution was incubated at 65°C for 10 minutes. Thereafter, the cover plate and the electrodes were removed and washed.

### Detection of Fluorescence

Fluorescences from Cy5 and Cy3 were measured using the same optical system as in Example 2.

By these methods, the fluorescences from the two types of the nucleic acid-immobilized sites were measured for Cy5 and Cy3. From the nucleic acid-immobilized site prepared by spotting the nucleic acid solution of TX803, fluorescence from Cy5 alone was observed, and fluorescence from Cy3 was not observed. From the nucleic acid-immobilized site prepared by spotting the nucleic acid solution of TX806, fluorescence from Cy3 alone was observed, and fluorescence from Cy5 was not detected.

The fluorescence intensities obtained from the nucleic acid-immobilized substrate subjected to the application of the AC voltage were comparable to those obtained by the conventional methods in which the hybridization is carried out for a long time, or in which a DC voltage is applied, even at a lower voltage.

### Example 9

To examine the effect of the case wherein the electrodes are not formed on the substrate for immobilizing selective binding substance, but provided to the apparatus for selective binding reaction, a slide glass (76mm x 26mm x 1mm) (produced by Matsunami Glass Kogyo) was cleaned with a mixed solution of pure water, ethanol and NaOH, and then washed with pure water. The cleaned surface was immersed in a mixed solution of pure water and poly-L-lysine (composition: 10% poly-L-lysine) to introduce amino groups to the surface of the slide glass. The immobilization of the nucleic acids and treatments of RNA were carried out in the same manner as in Example 8.

To confirm the effect of application of the AC voltage using the substrate for immobilizing nucleic acid, the following experiment was carried out. A substrate for immobilizing nucleic acid on which two types of nucleic acids were immobilized on the surface thereof was fixed on a base of the apparatus for hybridization, and electrodes connected to means for applying AC voltage of the apparatus for hybridization were arranged on both sides of the region in which the nucleic acids were immobilized. In this example, gold thin plates having a thickness of 0.15 mm were used as the electrodes. The distance between the electrodes was 1 cm. On the sites on which the two types of nucleic acids were immobilized, 20 µl of the above-described solution for hybridization was dropped, and a cover plate bridging the electrodes on both sides of the nucleic acid-immobilized sites was placed. The cover plate was closed tightly so as not to allow evaporation of the solution for hybridization. An AC voltage of 10V, 10Hz was applied between the electrodes and the solution was incubated at 65°C for 10 minutes. Thereafter, the cover plate and the electrodes were removed and washed.

For comparison with a conventional method in which AC voltage is not applied, as a sample to be compared with the sample to which AC voltage was applied, a sample obtained after incubation at 65°C for 16 hours without applying a voltage between the electrodes was provided. After incubation at 65°C for 16 hours, the cover plate and the electrodes were removed, and washed.

### Detection of Fluorescence

By the same method as in Example 8, the fluorescences from Cy5 and Cy3 were measured. From the nucleic acid-immobilized site prepared by spotting the nucleic acid solution of TX803, fluorescence from Cy5 alone was observed, and fluorescence from Cy3 was not observed. From the nucleic acid-immobilized site prepared by spotting the nucleic acid solution of TX806, fluorescence from Cy3 alone was observed, and fluorescence from Cy5 was not detected.

Further, the fluorescence intensities obtained from the nucleic acid-immobilized substrate with which the AC voltage was applied were about the same as those obtained when the electrodes were provided to the apparatus for hybridization.

## Claims

1. A fiber on which a selective-binding substance is immobilized, or a fiber array comprising a bundle of said fibers.

2. The fiber or the array comprising a bundle of said fibers according to claim 1, wherein said selective-binding substance is a nucleic acid, protein, saccharide, or an antigenic compound.

3. The fiber or the array comprising a bundle of said fibers according to claim 1, wherein said selective-binding substance is a nucleic acid, antibody or an antigen.

4. The fiber or the array comprising a bundle of said fibers according to claim 1, wherein said fiber transmits light.

5. The fiber and the array comprising a bundle of said fibers according to claim 1, wherein said fiber is electrically conductive.

6. The fiber and the array comprising a bundle of said fibers according to claim 1, wherein said fiber is flexible.

7. The fiber and the array comprising a bundle of said fibers according to claim 1, wherein said selective-binding substance is immobilized on an end of said fiber.

8. The fiber array according to claims 1 to 7, wherein all of said fibers or a part of said fibers in said fiber array carry different types of said selective binding substance.

9. The fiber array according to claim 8, wherein said fibers on which said selective binding substances are immobilized can be distinguished from each other.

10. The fiber array according to any one of claims I to 9, wherein a substance reacted with said immobilized selective binding substance can be directly detected through said fiber.

11. The fiber array according to any one of claims 1 to 9, wherein said fiber is an optical fiber.

12. A method for measuring the result of a selective binding reaction, comprising treating said fiber array according to any one of claims 1 to 11 with a test sample containing a corresponding selective binding substance which selectively binds to said selective binding substance immobilized on said fiber in said fiber array, and measuring said corresponding selective binding substance bound to said fiber after washing the fibers.

13. The method according to claim 12, wherein said fiber transmits light, and said selective binding substance is immobilized on an end of said fiber.

14. The method according to claim 13, wherein said corresponding selective binding substance is labeled with a fluorescent or luminescent substance, or is one which selectively binds to a substance for measurement labeled with a fluorescent or luminescent substance, and after reaction and washing, measuring the result of the reaction by measuring the fluorescence or luminescence from said label attached to said corresponding selective binding substance or said substance for measurement from another end of said fiber.

15. A method for selectively binding said selective binding substance immobilized on said fiber in said fiber array according to claim 5 with a test sample containing a corresponding selective binding substance, comprising applying electric voltage or electric current to said fiber array according to claim 5.

16. A method for binding reaction between a selective binding substance and a corresponding selective binding substance, comprising immobilizing said selective binding substance on a substrate; and making a test sample contacted to said immobilized selective binding substance, which test sample contains said corresponding selective binding substance which selectively binds to said selective binding substance, thereby carrying out said binding reaction; said test sample and/or said corresponding selective binding substance being moved relative to the surface on which said selective binding substance is immobilized, in said step of immobilizing said selective binding substance on a substrate and making a test sample contacted to said immobilized selective binding substance.

17. The method according to claim 16, wherein said binding reaction is carried out while applying an AC voltage in a direction crossing the perpendicular axis of the surface on which said selective binding substance is immobilized, between electrodes arranged outside the both ends of the aren on which said selective binding substance is immobilized, in said step of immobilizing said selective binding substance on a substrate and making a test sample contacted to said immobilized selective binding substance, which test sample contains said corresponding selective binding substance which selectively binds to said selective binding substance, thereby carrying out said binding reaction

18. The method according to claim 16 or 17, wherein a plurality of areas each on which said selective binding substance is immobilized exist, and a selective binding substance-arrayed region in which said areas are arranged exists, and said electrodes are arranged outside said selective binding substance-arrayed region.

19. The method according to any one of claims 16 to 18, wherein said selective binding substance is at least one selected from the group consisting of nucleic acids, proteins, saccharides, antibodies and antigenic compounds.

20. The method according to claim 19, wherein said selective binding substance and said corresponding selective binding substance are single-stranded nucleic acids, and said binding reaction is hybridization between the nucleic acids.

21. An apparatus for conducting a binding reaction including a step of immobilizing a selective binding substance on a substrate and making a test sample contacted to said immobilized selective binding substance, which test sample contains a corresponding selective binding substance which selectively binds to said selective binding substance, thereby carrying out said binding reaction, said apparatus comprising a base on which said substrate is mounted; electrodes arranged in a direction crossing the perpendicular axis of the surface on which the selective binding substance is immobilized, and arranged outside the both ends of the area on which said selective binding substance is immobilized; and means for applying AC voltage between said electrodes.

22. A substrate for conducting a binding reaction including a step of immobilizing said selective binding substance on said substrate and making a test sample contacted to said immobilized selective binding substance, which test sample contains said corresponding selective binding substance which selectively binds to said selective binding substance, thereby carrying out said binding reaction between said selective binding substance and said corresponding selective binding substance, said substrate comprising a site for immobilizing said selective binding substance, on which said selective binding substance on said substrate is immobilized; and electrodes arranged outside the both ends of region for immobilizing said selective binding substance.

23. The substrate according to claim 22, wherein a plurality of sites for immobilizing said selective binding substance exist, and a selective binding substance-arrayed region in which said sites are arranged exists, and said electrodes are arranged outside said selective binding substance-arrayed region.

24. The substrate for immobilizing the selective binding substance according to claim 22 or 23, **characterized in that** said site for immobilizing said selective binding substance is a protruded or hollow site.

25. The substrate according to claim 24, wherein said site for immobilizing said selective binding substance is an end of a fiber or a bundle of fibers, which fiber or bundle is inserted through a hole formed in said substrate.

26. The substrate according to any one of claims 22 to 25, wherein said selective binding substance is immobilized on said site for immobilizing said selective binding substance.

27. The substrate according to any one of claims 22 to 26, wherein said selective binding substance is at least one selected from the group consisting of nucleic acids, proteins, saccharides, antibodies and antigenic compounds.

28. The substrate according to claim 27, wherein said selective binding substance and said corresponding selective binding substance are single-stranded nucleic acids, and said binding reaction is hybridization between the nucleic acids.

29. The substrate according to any one of claims 22 to 28, wherein said electrodes are made of at least one material selected from the group consisting of simple metals selected from platinum, gold, silver, aluminum, copper and palladium, alloys thereof, carbon and carbon compounds, and electrically conductive polymers.
